# EUROPEAN PATENT APPLICATION

(11) **EP 0 528 515 A2**
(43) Date of publication of application: **24.02.1993**
(21) Application number: 92305045.4
(22) Date of filing: 02.06.1992
(51) Int. Cl.: A61K 31/40, A61K 31/365, A61K 31/22

(54) **HMG-CoA reductase inhibitors in the prevention of restenosis following coronary angioplasty**

(30) Priority: 04.06.1991 US 709940
(71) Applicant: MERCK & CO. INC., Rahway New Jersey 07065-0900 (US)
(72) Inventor: Boccuzzi, Stephen J., Long Valley, NJ 07853 (US); Hirsch, Laurence J., Westfield, NJ 07090 (US)
(74) Representative: Thompson, John Dr.

(57) **Abstract**

Treatment of coronary patients scheduled to undergo percutaneous transluminal coronary angioplasty (PTCA) for several days before the procedure and a prolonged period afterwards with doses of a HMG-CoA reductase inhibitor to aggressively lower total and LDL cholesterol prevents intimal hyperplasia at the angioplasty site reducing clinical and angiographic evidence of restenosis.

## Description

### BACKGROUND OF THE INVENTION

Percutaneous transluminal coronary angioplasty (PTCA) was introduced 13 years ago for the non-surgical treatment of coronary artery disease. The procedure currently enjoys an initial success rate of approximately 90% and serious complications requiring emergent coronary artery bypass surgery occur in less than 4% of cases. Despite good initial success and few complications, a physiologically significant stenosis reoccurs between one and six months at the site of angioplasty in approximately 30 to 35% of patients undergoing PTCA, and continues to be the main problem associated with this procedure. This rate of restenosis has remained constant despite the continued improvement in both initial success rates and the rate of complications. Numerous trials have been performed without success and many others are underway in an attempt to reduce the rate of post-PTCA restenosis.

To date, pharmacological intervention directed toward inhibiting restenosis have been largely unsuccessful including corticosteroids, antiplatelet agents, calcium channel blockers, anticoagulant therapy, and fish oil preparations.

There are data to indicate that there are similarities between native coronary artery lesions and those induced by balloon injury and that hyperlipidemia may contribute to the smooth muscle cell proliferation responsible for restenotic lesions.

Animal studies have demonstrated a beneficial effect of lovastatin in reducing the incidence of post angioplasty restenosis. In one study using atherosclerotic hypercholesterolemic rabbits, lovastatin administered post-angioplasty significantly decreased intimal hyperplasia in femoral arteries following balloon angioplasty (1) (2).

Another study evaluating the effect of lovastatin on rat carotid arteries post-PTCA demonstrated that pre-treatment 7 days with lovastatin before PTCA significantly reduced the formation of neointimal plaques (3).

Because of species differences between rabbit, rat and human; the difference between carotid, femoral and coronary arteries; and the difference in doses of lovastatin employed in the animal studies, the relevance to the present invention is not clear.

There have been numerous basic and animal studies demonstrating efficacy of various agents in the prevention of restenosis (4,5,6), but these results have not been confirmed in human clinical trials (7,8,9,10).

In a recent clinical study evaluating the effect of lovastatin 20-40 mg/day vs placebo immediately after PTCA, restenosis rates were significantly decreased in the lovastatin group, but angiographic followup was incomplete in both treatment groups (11). Another open clinical study evaluating the effects of lovastatin/colestipol therapy begun the day of PTCA failed to demonstrate any difference in the restenosis rate (12). A third study evaluating late regression of lesions beyond 6 months post-PTCA after aggressive treatment with lovastatin + colestipol, niacin + colestipol versus placebo revealed that late regression of residual stenosis in post-PTCA lesions was greater in frequency and magnitude in patients taking intensive lipid-altering therapy. This suggests that intensive lipid-lowering therapy might retard the restenosis process (13). However, the relevance of this study to the prevention of restenosis in the first 6 months is doubtful. None of these clinical studies utilized a pre-treatment period prior to vessel injury with balloon angioplasty, and lipid lowering may not have been aggressive enough to prevent restenosis.

Now, with the present invention, there is provided a method of reducing post-angioplasty restenosis comprising a pretreatment and post-treatment of coronary patients with of a HMG-CoA reductase inhibitor.

### REFERENCES

1. Gellman J, Ezekowitz MD; Lovastatin, a HMG CoA reductase inhibitor, reduces restenosis following balloon angioplasty in an atherosclerotic rabbit. Clin Res 37(2): 261A, 1989.
2. Gellman J, Ezekowitz M, Sarembock I, Azrin M, Nochomowitz L, Lerner E, Haudenschild C; Effect of lovastatin on intimal hyperplasia after balloon angioplasty: A study in an atherosclerotic hypercholesterolemic rabbit. J of the Amer Col of Cardiology 17(1): 251-259, 1991.
3. Volker W, Bruning T, Faber V, Eckardt H; Lovastatin reduces neointimal plaque formation of ballooned rat carotids. Third International Symposium: Treatment of Severe Dyslipoproteinemia in the Prevention of Coronary Heart Disease. Munich, October 18-19 1990.
4. Faxon DP, Sanborn TA, Haudenschild CC, Ryan TJ; Effect of antiplatelet therapy on restenosis after experimental angioplasty. Am. J of Card 53 (12) 72C-76C.
5. Gordon JB, Berk BC, Bettmann AP, et al; Vascular smooth muscle proliferation following ballooning injury is synergistically inhibited by low molecular weight heparin and hydrocortisone. Circulation 1987;76:IV-213.
6. Weiner BH, Ockene IS, Levine PH et al; Inhibition of antherosclerosis by cod-liver oil in a hyperlipidemic swine model. New England Journal of Medecine 315:14, 841-6.
7. Schwartz L, Lesperance J, Bourassa M et al; The role of antiplatelet agents in modifying the extent of restenosis following percutaneous transluminal coronary angioplasty. American Heart Journal 1990; 119-232.
8. Pepine CJ, Hirschfield JW, MacDonald RG et al; A controlled trial of corticosteroids to prevent restenosis after coronary angioplasty. Circulation 1990:81:1753-1761.
9. Ellis SG, Roubin GS, Wilentz J, Douglas JS, King SB; Effect of 18-24 hour heparin administration for prevention of restenosis after uncomplicated coronary angioplasty. Am Heart Journal 1989:117:777-782.
10. Reis GJ, Boucher TM, Sipperly ME, Silverman DI et al; Randomized trial of fish oil for prevention of restenosis after coronary angioplasty. Lancet 1989;177-181.
11. Sahni R, Maniet AR, Voci G, Banka V; Prevention of restenosis by lovastatin. Circulation (suppl II) 80: II-262, 1989.
12. Hollman J, Konrad K, Raymon R, Whitlow P, et al; Lipid lowering for the prevention of recurrent stenosis following coronary angioplasty. Circulation (suppl II) 80: II-261, 1989.
13. Zhao X, Flygenring B, Stewart D, Albers J, Bisson B, Bardsley J, Brown G; Increased potential for regression of post-PTCA restenosis using intensive lipid-altering therapy: Comparison with matched non-PTCA Lesions. J of Am Col of Cardiology 17:2 230 A, 1991.

### DETAILED DESCRIPTION OF THE INVENTION

The novel method of treatment of this invention comprises the administration to a patient scheduled to undergo percutaneous transluminal coronary angioplasty (PCTA) with a HMG-CoA reductase inhibitor for about 24 hours to 10 days, preferably about 3-10 days and even more preferably about 7-10 days before the PCTA procedure and for a period of time of about 6-12 months after the PCTA procedure, whereby restenosis is inhibited.

In the novel method of this invention the patient is a human scheduled for PTCA of a target stenotic lesion(s) in an artery or bypass graft.

The HMG-CoA reductase inhibitor for use in the novel method is preferably lovastatin, fluvastatin, simvastatin or pravastatin, especially lovastatin.

The doses of HMG-CoA reductase inhibitor contemplated for use in this invention are about 10 to 80 mg per day preferably given in single or divided doses and titrated to maintain LDL-cholesterol levels below about 100 mg /dl but not less than about 50 mg/dl.

## Claims

1. The use of a HMG-CoA reductase inhibitor for the manufacture of a medicament for inhibiting restenosis following percutaneous transluminal coronary angioplasty.

2. The use as claimed in Claim 1 wherein the dose of HMG-CoA reductase inhibitor is about 10 to 80 mg/day.

3. The use as claimed in Claim 2 wherein the HMG-CoA reductase inhibitor is lovastatin, fluvastatin or simvastatin.

4. The use as claimed in Claim 3 wherein the HMG-CoA reductase inhibitor is lovastatin.
